# EUROPEAN PATENT SPECIFICATION

(11) **EP 2 938 263 B1**
(45) Date of publication and mention of the grant of the patent: **15.05.2019**
(21) Application number: 13866939.5
(22) Date of filing: 17.12.2013
(51) Int. Cl.: A61B 8/12

(54) **FIRE CONTROL SYSTEM FOR ROTATIONAL IVUS**
AUSLÖSEKONTROLLSYSTEM FÜR ROTIERENDE IVUS
SYSTÈME DE COMMANDE DE TIR POUR SYSTÈME D'IMAGERIE ULTRASONORE INTRAVASCULAIRE ROTATIF

(30) Priority: 27.12.2012 US 201261746532 P
(43) Date of publication of application: 04.11.2015
(73) Proprietor: Volcano Corporation, San Diego, CA 92130 (US)
(72) Inventor: CORL, Paul Douglas, Palo Alto, CA 94306 (US)
(74) Representative: de Haan, Poul Erik
(86) International application number: PCT/US2013/075755
(87) International publication number: WO 2014/105526

(56) References cited:
- JP-A- 2009 072 597
- US-A- 5 125 410
- US-A- 5 345 938
- US-A- 5 377 682
- US-A- 5 423 740
- US-A- 6 013 033
- US-A1- 2007 244 391
- US-A1- 2012 017 013
- US-A1- 2012 035 596
- US-A1- 2012 172 727

## Description

### TECHNICAL FIELD

The present disclosure relates generally to intravascular ultrasound (IVUS) imaging inside the living body and, in particular, to a control system for an IVUS imaging system using a rotational catheter that relies on a mechanically-scanned ultrasound transducer.

### BACKGROUND

Intravascular ultrasound (IVUS) imaging is widely used in interventional cardiology as a diagnostic tool for a diseased vessel, such as an artery, within the human body to determine the need for treatment, to guide the intervention, and/or to assess its effectiveness. IVUS imaging uses ultrasound echoes to create an image of the vessel of interest. The ultrasound waves pass easily through most tissues and blood, but they are partially reflected from discontinuities arising from tissue structures (such as the various layers of the vessel wall), red blood cells, and other features of interest. The IVUS imaging system, which is connected to the IVUS catheter by way of a patient interface module (PIM), processes the received ultrasound echoes to produce a cross-sectional image of the vessel where the catheter is placed.

In a typical rotational IVUS catheter, a single ultrasound transducer element is located at the tip of a flexible driveshaft that spins inside a plastic sheath inserted into the vessel of interest. The transducer element is oriented such that the ultrasound beam propagates generally perpendicular to the axis of the catheter. A fluid-filled sheath protects the vessel tissue from the spinning transducer and driveshaft while permitting ultrasound signals to freely propagate from the transducer into the tissue and back. As the driveshaft rotates (typically at 30 revolutions per second), the transducer is periodically excited with a high voltage pulse to emit a short burst of ultrasound. The same transducer then listens for the returning echoes reflected from various tissue structures, and the IVUS imaging system assembles a two dimensional (2D) display of the vessel cross-section from a sequence of these pulse/acquisition cycles occurring during a single revolution of the transducer. In order to form an accurate image, free from geometric distortion, the transducer angle must be accurately known for each pulse/acquisition cycle. This is challenging in the face of variations in drag forces on the catheter, irregularities in the motor motion, and other factors that tend to disrupt the phase relationship between the rotation of the transducer in the tip of the catheter and the rotation of the motor shaft.

Traditional rotational IVUS systems use a high resolution rotary encoder (typically 512 pulses per revolution) mounted on the motor shaft to subdivide one rotation of the catheter driveshaft into, for example, 512 pulse/acquisition sequences with nominally uniform angular spacing. This approach relies on the assumption that the angular position of the motor/encoder accurately represents the angular position of the transducer mounted at the tip of the flexible driveshaft. Variable drag and torsional asymmetries in the flexible driveshaft may give rise to geometric distortion in the image, commonly referred to as Non-Uniform Rotational Distortion (NURD), when the correlation between motor angle and transducer angle is disrupted. Separately, a motor control circuit maintains the motor speed at the desired nominal value (typically 30 rotations per second). Typically, rotational IVUS systems rely on small brushless DC motors, electronically commutated to maintain high efficiency and maximum torque, and using encoder feedback to maintain the desired average rotational speed.

Using traditional approaches pulse/acquisition cycles triggered by the encoder output are synchronized to the motor rotation, and to the extent that the motor speed varies, the intervals between pulse/acquisition cycles vary as well. While existing IVUS catheters deliver useful diagnostic information, there is a need for enhanced image quality to provide more valuable insight into the vessel condition. For further improvement in image quality in rotational IVUS, it is desirable to improve transmit electronics or other signal processing advances.

Accordingly, there remains a need for improved devices, systems, and methods for providing synchronized signals in an intravascular ultrasound imaging system.

### SUMMARY

According to embodiments disclosed herein a patient interface module (PIM) for use in an intra-vascular ultrasound imaging (IVUS) system as defined in claim 1, is provided.

According to some embodiments, an imaging system may include a monitor; a processing system; a patient interface module (PIM) as defined in claim 1; and a catheter coupled to the PIM, the catheter including a transducer.

According to some embodiments a method for producing synchronized signals in a fire control system for an intra-vascular ultrasound (IVUS) imaging system as defined in claim 11, is provided.

These and other embodiments of the present invention will be described in further detail below with reference to the following drawings.

### BRIEF DESCRIPTION OF THE DRAWINGS

FIG. 1 is a schematic view of an imaging system, according to some embodiments of the present disclosure.
FIG. 2A shows a partial schematic view of a Patient Interface Module (PIM) for use in an IVUS imaging system, according to some embodiments of the present disclosure.
FIG. 2B shows a partial schematic view of a Patient Interface Module (PIM) for use in an IVUS imaging system, according to some embodiments of the present disclosure.
FIG. 3A shows a partial view of synchronized signals from a synchronous PIM, according to some embodiments of the present disclosure.
FIG. 3B shows a partial view of a synchronized echo received by a synchronous PIM, according to some embodiments of the present disclosure.
FIG. 4A shows a partial view of a motor, according to some embodiments of the present disclosure.
FIG. 4B shows a partial view of a multi-phase motor drive waveform, according to some embodiments of the present disclosure.
FIG. 5 shows a partial view of a clock and timing circuit to produce synchronized signals, according to some embodiments of the present disclosure.
FIG. 6 shows a partial view of a flow chart in a method for producing synchronized signals in a fire control system for rotational IVUS.

In the figures, elements having the same reference number have the same or similar functions.

### DETAILED DESCRIPTION

For the purposes of promoting an understanding of the principles of the present disclosure, reference will now be made to the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described may be combined. For the sake of brevity, however, the numerous iterations of these combinations will not be described separately.

A patient interface module (PIM) and a method for controlling the motor speed and the scan line triggering in a rotational IVUS imaging system are provided. A scan line triggering includes transmit pulse generation and echo signal data acquisition from a rotating transducer at the distal end of a catheter. Due to torsion flexibility of the driveshaft of catheter 102, the transducer position may deviate quite significantly from the motor position, creating non-uniform rotational distortion (NURD) artifacts. NURD artifacts are exacerbated by asymmetrical bending moments of the driveshaft that cause variances in the rotational speed of the driveshaft.

The traditional approach is to use a high resolution rotary encoder coupled to the motor drive. In some embodiments, control of the motor speed and scan line triggering is performed using a synchronous motor drive. In a synchronous motor drive stalling of the motor may be a problem if too much drag is reached, which is desirable in medical applications. Synchronous motor drives include added safety due to torque limitation. Embodiments consistent with the present disclosure may operate in open loop control: a synchronous drive of the rotary motor at a desired speed is controlled electronically and monitored. In some embodiments, a brushless DC (BLDC) with motor speed feedback may be used. A speed feedback attempts to maintain the motor at a fixed speed, enhancing efficiency. A BLDC approach can deal with torque variations, but has variable speed. In some embodiments a low resolution sensor in the motor may be coupled to a highly sensitive clock and timing circuit. In some embodiments, the low resolution sensor in the motor is a group of Hall-effect sensors. According to some embodiments a synchronous motor drive and synchronous scan line trigger circuitry is used, eliminating the need for a high resolution rotary encoder coupled to the motor drive.

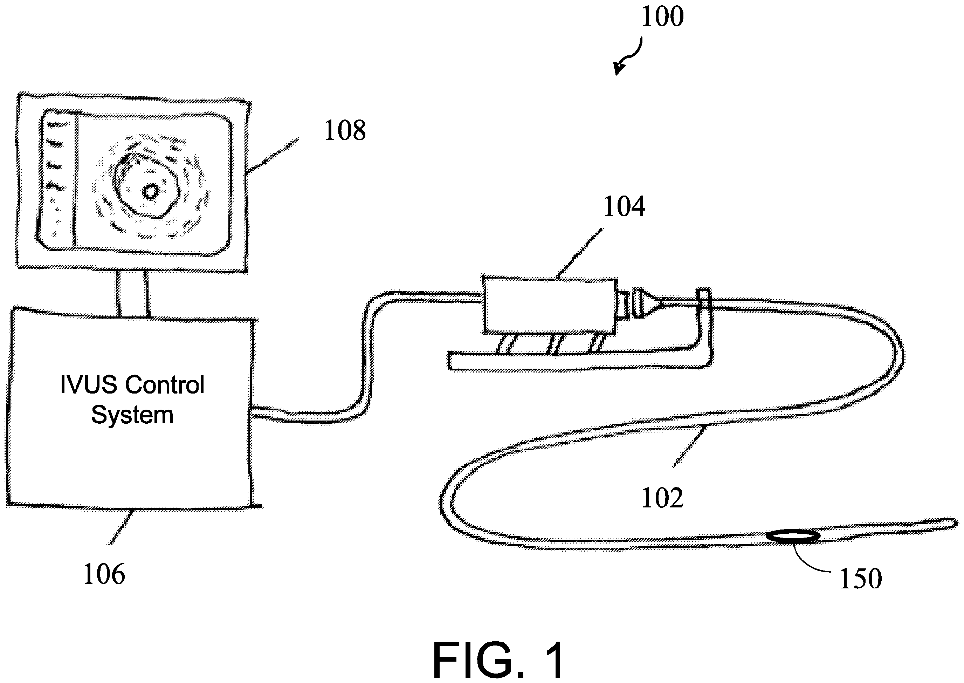

FIG. 1 shows an IVUS imaging system 100 according to an embodiment of the present disclosure. The IVUS imaging system 100 is a rotational IVUS imaging system. In that regard, the main components of the rotational IVUS imaging system are a rotational IVUS catheter 102, a patient interface module (PIM) 104, an IVUS console or processing system 106, and a monitor 108 to display the IVUS images generated by the IVUS console 106. Catheter 102 includes an ultrasound transducer 150 according to some embodiments. PIM 104 implements the appropriate interface specifications to support catheter 102. According to some embodiments, PIM 104 generates a sequence of transmit trigger signals and control waveforms to regulate the operation of ultrasound transducer 150.

Ultrasound transducer 150 transmits ultrasound signals to the vessel tissue in response to the trigger signals received from PIM 104. Ultrasound transducer 150 also converts echo signals received from the vessel tissue and/or other surrounding structures into electrical signals that are communicated to console 106 via PIM 104. Ultrasound echo signals received by PIM 104 in response to a single ultrasound transmit pulse may be used to form a line scan (A-scan) of a target tissue depth along an axial direction relative to a longitudinal axis (LA) of the catheter. In some embodiments, PIM 104 also supplies high- and low-voltage DC power supplies. A high voltage may be up to 80V, and typically including voltages between 60-70 V. In some embodiments, the voltage provided by PIM 104 may be as low as 3.3 V. Accordingly, in some embodiments such as a PMUT transducer an application specific integrated circuit (ASIC) may be used to provide a voltage to the transducer. In some embodiments the ASIC may be included in the distal portion of the catheter, and in some embodiments part of the ASIC may be included in PIM 104. For example, the ASIC may use the DC provided by PIM 104 to generate a higher-voltage pulse for a short period of time. In some embodiments the higher-voltage pulse may be up to 120 V, 150 V, and even higher, lasting for a few nanoseconds (1 nanosecond, Ins = 10⁻⁹ s). The voltage provided by PIM 104 ultimately is delivered to the distal end of rotational IVUS catheter 102.

Furthermore, for some catheters, such as those described in US Patent Application No. 8,104,479, 5,243,988, and 5,546,948, PIM 104 simply transmits high-voltage signals directly to transducer 150. For example, the voltage may be as high as 400 V peak-to-peak on a cycle that lasts a few ns (each cycle resembles a waveform having symmetric positive voltage and negative voltage periods). In some embodiments, PIM 104 delivers a DC voltage to transducer 150 across a rotational interface. In that regard, options for delivering DC power across a rotating interface include the use of slip-rings, and/or the implementation of active spinner technology.

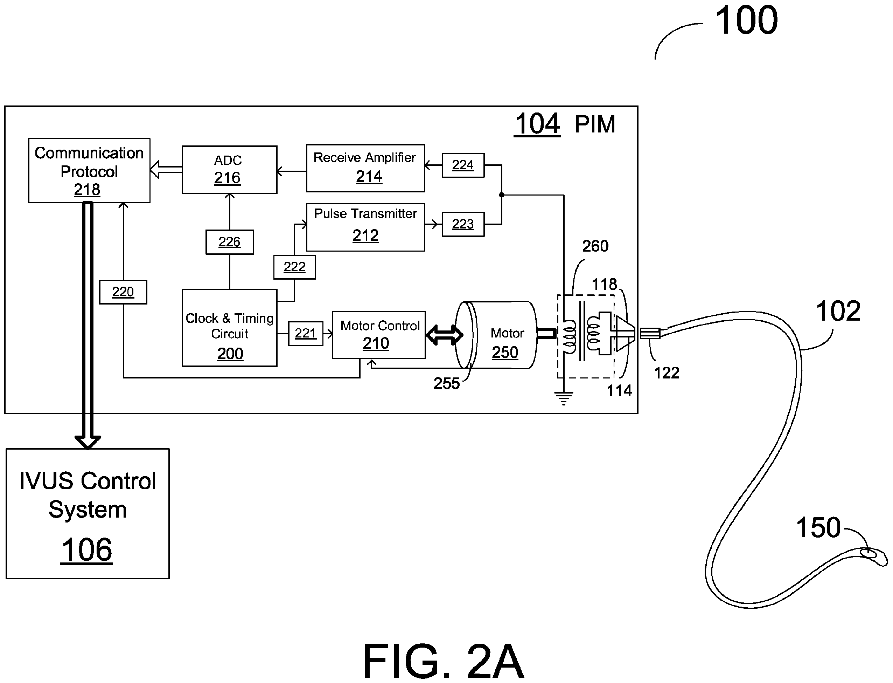

FIG. 2A shows a partial schematic view of a PIM 104 for use in IVUS imaging system 100, according to some embodiments of the present disclosure. FIG. 2A illustrates PIM 104 in more detail, including a motor 250 to provide rotational motion to transducer 150 in the distal portion of catheter 102 through shaft 114 that extends along the length of the catheter. Shaft 114 is attached to PIM 104 by a connector 118 that fits into a telescope 122. Telescope 122 allows the length of catheter 102 to be adjusted. PIM 104 also includes a rotary transformer 260 to provide pulse signal 223 to transducer 150. Rotary transformer 260 also transmits electrical signals 224 from transducer 150 to PIM 104. Pulse signal 223 is provided by pulse transmitter 212, and electrical signals 224 are amplified by receive amplifier 214. According to some embodiments, electrical signal 224 is an analog signal including an echo response from the vessel tissue and/or other surrounding structures as detected by transducer 150. Analog-to-digital converter (ADC) 216 converts amplified electrical signal 224 into a digital signal that is transferred out of PIM 104 to IVUS control system 106 by communication protocol circuit 218.

Motor 250 is an electric motor, such as a brushless motor, in some implementations. In some embodiments, motor 250 is a brushless DC motor. 2-phase or 3-phase motor with permanent magnet rotor. In some embodiments, motor control 210 is a circuit that controls the spin speed of motor 250. In that regard, motor control 210 is configured to cause the motor 250 to rotate at a desired rotational speed, which is about 30 revolutions per second in some embodiments. The motor speed is precisely controlled by synchronous motor drive signals 221 provided by clock and timing circuit 200. The motor torque and phase are controlled by control circuit 210.

Use of a low resolution position sensor in the motor coupled to clock and timing circuit 200 according to embodiments disclosed herein, provides a well-defined motor speed. Clock and timing circuit 200 reduces the vulnerability of a rotational IVUS catheter to enter a runaway rotational velocity under a fault condition by continuously adjusting the phase and frequency of the motor rotation. A synchronous control provided by clock and timing circuit 200 ensures precise motor speed with a simplified timing control. According to some embodiments, clock and timing circuit 200 provides transmitter timing signal 222 and motor speed control signal 221 using a common stable system clock.

In some embodiments, PIM 104 has a hardware structure that eliminates the need for a high resolution encoder in motor 250. Some embodiments include a rotation signal 220 provided by motor 250 to motor control circuit 210 and communication protocol circuit 218. Signals 221, 222, and 226 are synchronous to one another while rotation signal 220 may have a phase delay, indicative of drag or some asynchronous behavior of motor 250.

Some embodiments include a rotational IVUS imaging systems as system 100. Some embodiments consistent with the present disclosure may include any type of transducer 150, for example traditional PZT devices, PMUT devices, CMUT devices, and/or combinations thereof. In some embodiments, the transducer 150 is replaced with an optical element (e.g., mirror, prism, and/or other reflector or emitter), such as those used in intravascular optical coherent tomography (OCT) imaging. In that regard, in a PMUT device a portion of pulse transmitter 212 and a portion of receive amplifier 214 may be included in an application specific integrated circuit (ASIC) at the distal end of catheter 102, proximal to transducer 150.

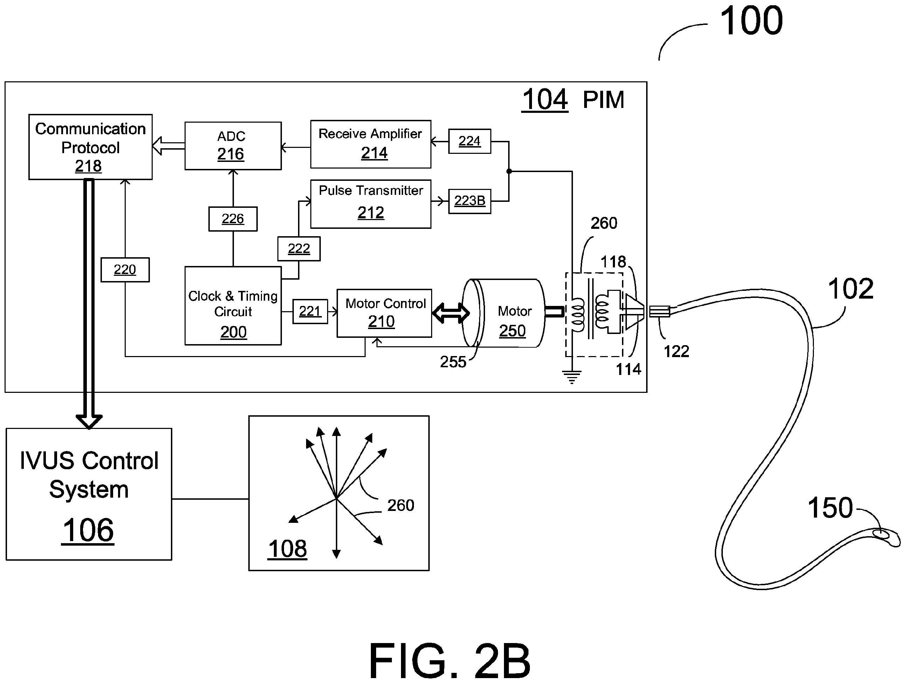

FIG. 2B shows a partial schematic view of a Patient Interface Module (PIM) 104 for use in an IVUS imaging system, according to some embodiments of the present disclosure. Accordingly, FIG. 2B includes catheter 102 having transducer 150, and IVUS control system 106 as described in detail above (cf. FIG. 2A). PIM 104 in FIG. 2B is also as described in reference to FIG. 2A. Accordingly, FIG. 2B shows a signal 223B provided by pulse transmitter 212 to transducer 150 in catheter 102. In some embodiments, signal 223B may include evenly spaced pulse triggers for transducer 150, irrespective of a phase delay of signal 220 from motor 250 relative to control signal 221. Thus, signal 223B may include triggers for evenly spaced ultrasound pulses to transducer 150 at a fixed rate. IVUS control system 106 may be able to adequately form a 2D scan image on display 108 by using knowledge of the phase delay signal 220 provided by communication protocol circuit 218. In that regard, while a plurality of ultrasound transmit pulses may be triggered at even time periods by pulse transmitter 212, A-scan lines 260 generated by each transmit pulse may be disposed at varying spatial directions by IVUS control system 106.

In some embodiments consistent with FIGS. 2A and 2B, motor control circuit 210 may be a synchronous control circuit providing a multi-phase control signal to motor 250. In some embodiments, motor control circuit 210 may include a brushless DC control circuit (BLDC) having a feedback loop and an encoder to more precisely control the speed of motor 250.

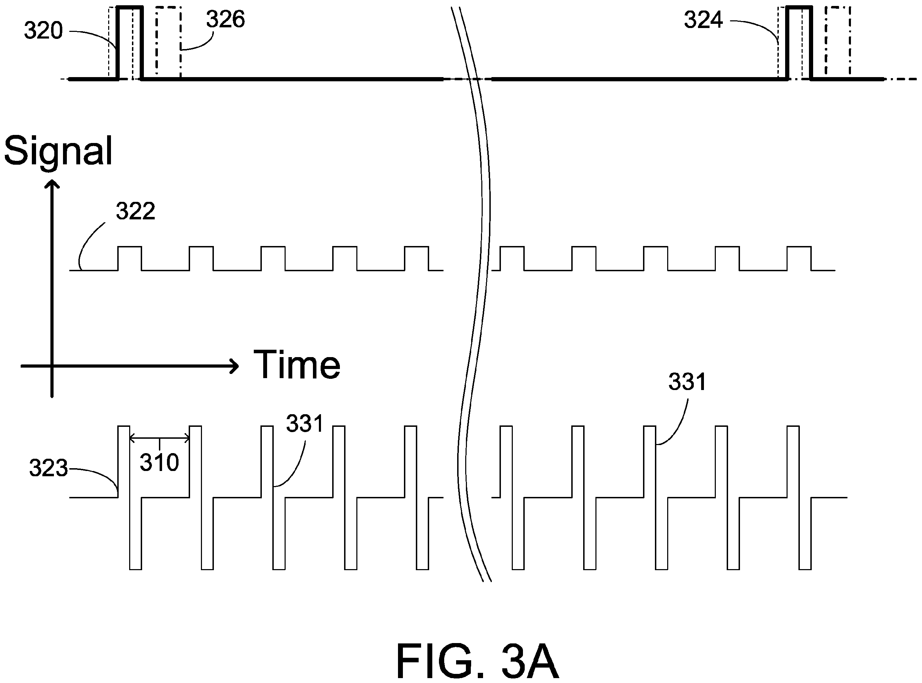

FIG. 3A shows a partial view of synchronized signals 320, 322, and 323 from synchronous PIM 104, according to some embodiments. The horizontal axis in FIG. 3A depicts time, and the vertical axis depicts a signal amplitude, which may be a voltage. Signal 320 is representative of a rotation signal, such as rotation signal 220, signal 322 is representative of a transmitter timing signal, such as transmitter timing signal 222, and signal 323 is representative of a pulse signal, such as pulse signal 223, all described in detail above in reference to FIG. 2. Accordingly, in some embodiments signal 320 is a low resolution rotation signal provided to clock and timing circuit 200 by motor control 210, using sensors in motor 250; signal 322 is a trigger signal provided by clock and timing circuit 200 to pulse transmitter 212 to trigger pulse signal 323; and pulse signal 323 is provided to transducer 150 to generate an ultrasound excitation impulse that propagates through the vessel tissue generating an echo signal.

FIG. 3A illustrates the low frequency of rotation signal 320 relative to
timing signal 322 and pulse signal 323. In some embodiments, the three signals 320, 322, and 323 are synchronous. In some embodiments, rotation signal 320 may shift somewhat in time relative to signals 322 and 323. For example, in some embodiments a rotation signal 326 may get ahead of signals 322 and 323, or a rotation signal 324 may lag behind signals 322 and 323. Embodiments consistent with the present disclosure may not force motor signal 326 or 324 into lockstep with signals 322 and 323, but rather record a precise value of the misalignment relative to signals 322 and 323. In embodiments using a BLDC motor controller 210 (cf. FIG. 2B) misalignment between motor signal 320 and signals 322 and 323 may be relatively small, since a BLDC controller may include a feedback loop to adjust motor speed and/or phase. In embodiments using a synchronous control of motor 250 in an open loop, the misalignment may be larger due to torsional asymmetries in the rotation of transducer 150. Rather than attempting to solve the misalignment by providing a control signal to motor 250, embodiments consistent with the present disclosure provide a record of the misalignment value for each A-scan, in order to generate an accurate 2D image through data processing at IVUS control system 106.

Transmitter timing signal 322 and pulse signal 323 may have the same frequency, according to some embodiments. As illustrated in FIG. 3A, the frequency of transmitter timing signal 322 and pulse signal 323 may be much higher than the frequency of rotation signal 320. In some embodiments, timing signal 322 has a frequency 512 times higher than the frequency of rotation signal 320. Thus, in some embodiments there may be 512 pulses in timing signal 322 equally spaced in time between two consecutive pulses in rotation signal 320. Other configurations are possible where the number of pulses between two pulses in rotation signal 320 are higher than 512 or lower than 512.

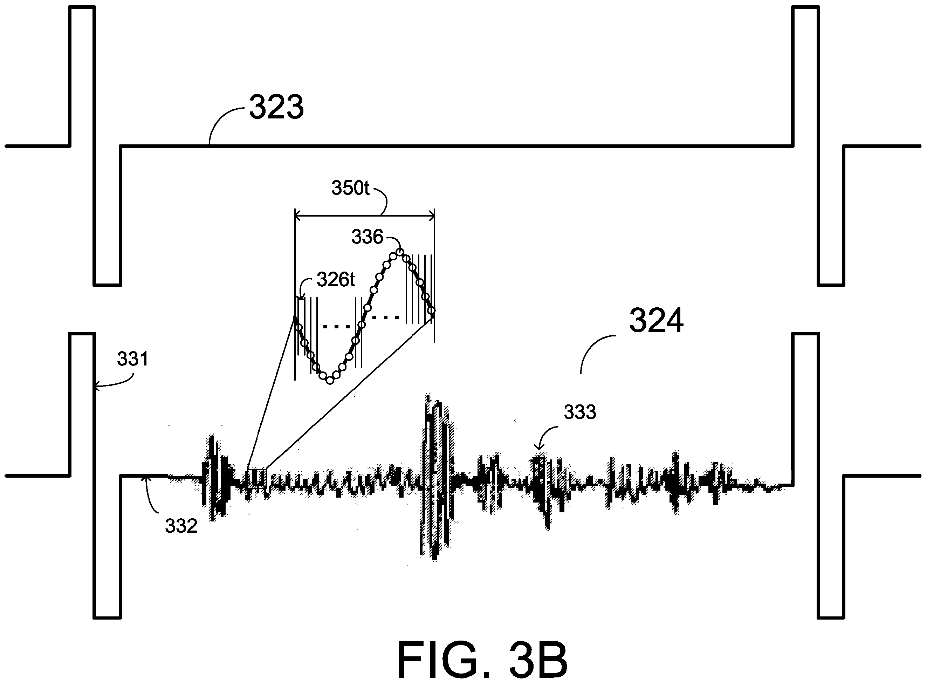

As shown in Fig. 3A, in some embodiments pulse signal 323 includes a train of pulses 331 having a signal profile including a negative amplitude for a period of time, immediately followed by a positive amplitude for the same period of time. The specific profile of pulse signal 323 is not limiting, and one of ordinary skill would recognize that other arrangements and configurations may be used. Pulse signal 323 generally has a higher amplitude than timing signal 322. Pulse signal is provided through catheter 102 to drive transducer 150 and produce an ultrasound impulse. The ultrasound impulse generates an echo signal in the vessel tissue, which propagates back to transducer 150 and produces an electrical signal such as signal 324, described in detail below in relation to FIG. 3B. Generally, a scan line of IVUS imaging system 100 is obtained from the echo signal during scan line interval 310, between two successive pulses 331 in signal 323.

FIG. 3B shows a partial view of a synchronized echo 324 received by synchronous PIM 104, according to some embodiments. Also illustrated in FIG. 3B is signal 323 with a pulse 331, for clarity. Synchronized echo 324 is provided to receive amplifier 214 in PIM 104, which may further amplify the signal prior to sending it to ADC 216. Portions of signal 323 are overlaid to echo 324 for illustrative purposes. Synchronized echo 324 includes a quiet portion 332 as the ultrasound propagates through a saline solution between transducer 150 and a sheath covering catheter 102. Quiet portion 332 carries no information regarding the vessel tissue or other surrounding structures. Synchronized echo 324 also includes tissue echo signal 333, which contains information from the vessel tissue and/or other surrounding structures, including biological and non-biological structures (e.g., stents).

FIG. 3B illustrates a blow up portion of tissue echo 333 showing a cycle or period of an ultrasound wave transmitted through the tissue. The ultrasound wave has an ultrasound period 350t which is associated to an ultrasound frequency 350f. In some embodiments, ultrasound frequency 350f is a center frequency of oscillation of transducer 150. For example, in some embodiments, ultrasound frequency 350f may be 40 MHz (Mega-Hertz, or 10⁶ Hz). Furthermore, tissue echo 333 may include signals having multiple frequencies within a bandwidth centered on frequency 350f. The bandwidth is given by a response function of transducer 150. Bandwidths of up to 75% or more of the nominal center frequency may be used. Some embodiments may have an ultrasound frequency 350f centered at 10 MHz, 20 MHz, 40 MHz, 60 MHz, 80 MHz, or higher values. Tissue echo 333 is amplified by receive amplifier 214 and provided to ADC 216. ADC 216 converts the analog electrical signal from tissue echo 333 into a digital signal by using a digitizing interval 326t between consecutive sampling points 336. Sampling points 336 are selected by ADC 216 from an amplified tissue echo 333.

In some embodiments, digitization interval 326t and the precise location of sampling points 336 is selected by ADC 216 using digitizing signal 226 (cf. FIG. 2). Accordingly, digitizing signal 226 is provided by clock and timing circuit 200 to ADC 216 by using a frequency multiple of rotation signal 220 or signal 320. In some embodiments, digitizing interval 326t is shorter than ultrasound period 350t. For example, in some embodiments digitizing interval 326t may be a few nanoseconds long (ns = 10⁻⁹ sec), while ultrasound period 350t is a few tens of ns. In some embodiments where ultrasound frequency 350f is centered at 40 MHz, the frequency 326f of a digitizing signal 226 corresponding to period 326t is approximately 160 MHz. Thus, according to some embodiments clock and timing circuit 200 provides signals 220, 226, and 221 at very different frequency scales.

Having a precise and evenly spaced timing between pulses 331 enables the use of advanced signal processing techniques including Doppler measurements. Thus, as the interval between successive pulses is evenly spaced, a precise control of the echo signal timing may be obtained avoiding interference between signals from subsequent transmit pulses. An evenly spaced time spacing of transmit pulses provides a constant baseline. Thus, a constant background level can be treated as a fixed artifact that can be removed utilizing standard signal processing.

In some embodiments the intervals between the transmit pulses is evenly spaced to within a small fraction of the ultrasound period 350t. For example, for a 40 MHz ultrasound signal, period 350t is 25 ns, so a clock and timing circuit as disclosed herein may provide transmit pulses 323 with stability better than 1 ns. For example, in Doppler signal processing it is often desirable to have precise intervals between transmit pulses 323, synchronized with echo signal 324. Thus, for Doppler signal processing the relative phase between corresponding data samples 336 in subsequent pulses 323 may be used to accurately determine tissue motion (blood, vessel wall contraction, etc.). In some embodiments advanced data processing algorithms are enabled or improved by the precise synchronization between signals 320 (220), 322 (220), 323 (223), and 324 (224). For example, some embodiments may use correlation processing between scan lines to support anti-NURD algorithms. In advanced correlation techniques it is desirable that the relative phase between successive scan lines be well known and precisely controlled, such as in embodiments consistent with the present disclosure. Other advanced processing techniques using high pulse repetition frequency (prf, e.g. the frequency of pulse signal 324) may benefit from a synchronous motor control as described above. Some of these techniques include synchronous data acquisition, noise reduction by signal averaging, dynamic range improvement algorithm, and pulse-inversion harmonic processing. Generally, data processing techniques that benefit from a high prf may be used in embodiments consistent with the present disclosure, since evenly spaced signaling schemes as described in FIGS. 3A and 3B enable the use of a high prf.

In some embodiments, use of evenly spaced trigger pulses enables the use of a pulse-inversion harmonic technique. Pulse-inversion harmonic is an advanced signal processing used in nonlinear acoustic measurements. Nonlinear acoustic effects may provide detailed information for tissue characterization. Pulse inversion harmonic addresses harmonic distortion by sending positive and negative pulses so that linear effects are canceled out (non-linearity acts as a squared function and therefore is not removed). In this regard, overlap of echo signals produced by subsequent pulses having the same phase is undesirable. Thus, as in Doppler measurements, timing precision of pulses and interference avoidance is relevant. For example, precision down to a few picoseconds (e.g., 10 picoseconds) is desirable. Some embodiments may provide 100 picoseconds or 1 nanosecond time precision in both Doppler measurement applications and in pulse-inversion applications.

Accordingly, an equal interval transmit 310 for all pulses 331, is desired irrespective of the motor positioning. For high level data analysis, it is desirable to have the maximum number of pulse-per-revolution (ppr) available. In order to avoid interference between transmit pulses 331 and the ultrasound echo signals, scan line interval 310 may be no less than the time it takes for a pulse to travel into about a 7 mm tissue depth, and back. In this regard, a fixed interval transmit 310 typically renders a larger number of ppr. In some embodiments, it is desirable that scan line interval be at least 10 µs. In that regard, the frequency of signal 323 may be about 100 KHz, which for a rotational speed of about 3000 pulses per revolution would yield about 3000 ppr, according to some embodiments.

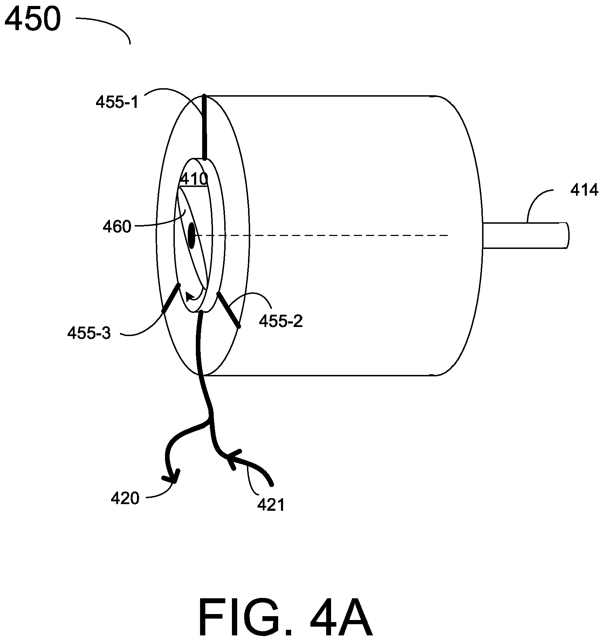

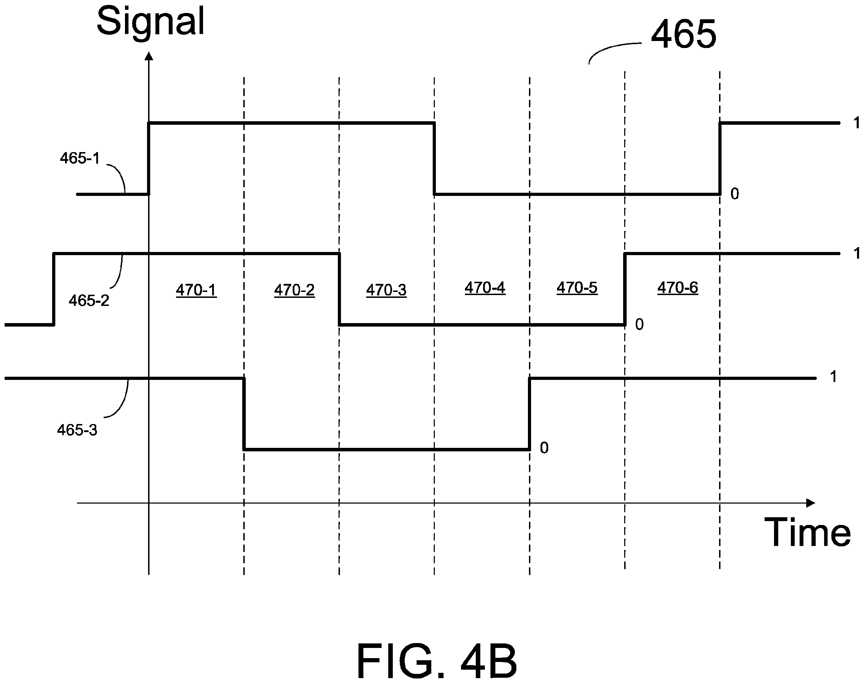

FIG. 4A shows a partial view of motor 450, according to some embodiments. Motor 450 includes a shaft 414, and a number of position sensors 455-1 through 455-3, built into the motor (collectively referred to as sensors 455). Also shown in FIG. 4A is motor control 410, which may be mounted on the exterior portion of motor 450 according to some embodiments. Motor 450, shaft 414, and motor control 410 may be as described in detail above in relation to motor 250, shaft 114, and motor control 210 (cf. FIG. 2). The precise number of sensors 455 included in motor 450 may vary according to the application, and is not limiting of the general concept embodied in FIG. 4. Sensors 455 detect the rotational state of shaft 414 in motor 450. In some embodiments, the position of shaft 414 may be indicated by a magnet 460 positioned with a center on shaft 414. Sensors 455 may include optical detectors and signals, or electromagnetic sensors and signals. For example, in some embodiments sensors 455 include at least one Hall-effect sensor that measures the magnetic field produced by opposite poles (e.g., North N, and South, S) in magnet 460.

Some embodiments may include encoders in sensors 455 providing better resolution than a hall effect sensor. While some embodiments may include a synchronous motor controller operating in open loop, a balance circuitry in motor control 410 may enable efficient operation, sufficient torque and avoid motor overheating. Embodiments using a BLDC controller with a feedback loop may use high resolution encoders to record exact motor position for each pulse.

FIG. 4A illustrates cables providing a speed control signal 421 to motor control 410, and delivering a rotation signal 420 from motor control 410. Speed control signal 421 may be as speed control signal 221 and rotation signal 420 may be as rotation signal 220, described in detail above in relation to FIG. 2. Thus, speed control signal 421 may be provided to motor control 410 by a clock and timing circuit such as clock and timing circuit 200. Rotation signal 420 may be provided by motor control 410 to communication protocol circuit such as communication protocol circuit 218 (cf. FIG. 2). Thus, IVUS control system 106 may perform data processing using an accurate value of the phase of rotor 414.

FIG. 4B shows a partial view of a multi-phase motor drive waveform 465, according to some embodiments of the present disclosure. Accordingly, waveform 465 includes three phases provided by waveform 465-1, 465-2, and 465-3. In that regard, waveform 465-1 may be provided by sensor 455-1, waveform 465-2 may be provided by sensor 455-2, as magnet 460 makes a complete turn in shaft 414. And waveform 465-3 may be provided by sensor 455-3. Waveforms 465 have the same or similar frequency and are separated in phase with respect to one another. As seen in FIG. 4B, waveforms 465 define six different phase configurations 470-1, 470-2, 470-3, 470-4, 470-5, and 470-6 (hereinafter collectively referred to as configurations 470) for a cycle of rotation of motor 450. Thus, by identifying the phase states of each sensor in multi-phase waveform 465, the position of shaft 414 may be determined with precision. Thus, in some embodiments multi-phase waveform 465 is included in signal 420 provided to communication protocol circuit 218. Likewise, multi-phase waveform 465 may be included in control signal 421 as a reference for motor controller 410, to determine a phase lag between the actual position of shaft 414 and a nominal position of shaft 414.

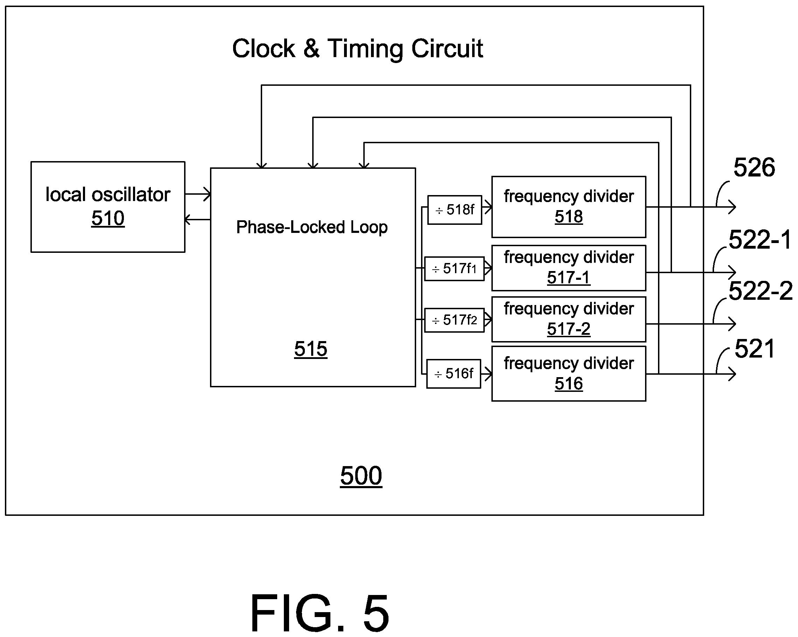

FIG. 5 shows a partial schematic view of a clock and timing circuit 500 to produce synchronized signals 521, 522, and 526 according to some embodiments of the present disclosure. Clock and timing circuit 500 includes a local oscillator 510, a phase-locked loop (PLL) 515, and frequency divider circuits 516, 517-1, 517-2, and 518. Local oscillator 510 may be a voltage controlled oscillator or a crystal oscillator. Frequency divider circuit 518 uses a frequency dividing factor 518f to provide a digitization clock signal 526 to ADC circuit 216. Likewise, frequency divider circuit 517-1 uses a frequency dividing factor 517f1 to provide a pulse-shaping signal 522-1 to pulse transmitter 212. For example, the pulse shaping signal may be a one-cycle profile at a nominal center frequency of the pulse signal. Frequency divider circuit 517-2 may use a frequency dividing factor 517f2 to provide a pulse repetition frequency (prf) 522-2 for pulse transmitter 212. For example, using pulse shaping signal 522-1 and prf 522-2, pulse transmitter 212 provides signal 223 to transducer 150 having a number of one-cycle pulses at a nominal center frequency, spaced at a fixed interval 310 (cf. FIGS. 2A-3A). Frequency divider circuit 516 may use a frequency dividing factor 516f to provide a multi-phase signal 521 to a motor such as motor 250 (cf. FIG. 2A) or motor 450 (cf. FIG. 4A).

As an illustrative example of embodiments of FIG. 5, crystal oscillator 510 may operate at a frequency approximately equal to 640 MHz. Then, a dividing factor 518f of about four (4) may result in an ADC clock signal 526 of about 160 MHz. In that regard, frequency dividing factor 517f1 may be cascaded from frequency dividing factor 518f to provide a further dividing factor of four, for a total dividing factor of sixteen (16) relative to 640 MHz. That is, signal 522-1 may be about 40 MHz (∼ 640 MHz/16), for a transducer 150 having a response frequency centered at about 40 MHz. Frequency dividing factor 517f2 may be cascaded from 517f1 by a further factor of 432 to result in prf 522-2 having a frequency of about 92.5 KHz (∼ 640 MHz/16/432). Furthermore, frequency dividing factor 516f may be cascaded down from frequency dividing factor 517f2 to provide a further dividing factor of about 3072 relative to 640 MHz. That is, signal 521 may be about 30.14 Hz (∼ 640 MHz/16/432/3072). Specific values of dividing factors and frequencies may vary according to the application without limitation to embodiments consistent with the present disclosure. One of ordinary skill will recognize that different frequencies and frequency dividing factors may be used at any stage in clock and timing circuit 500, consistent with embodiments disclosed herein.

Some embodiments include using a motor phase monitoring algorithm instead of controlling the motor speed directly. In such embodiments, a multi-phase waveform guarantees that the average motor speed is maintained at a nominal value (e.g., 30 revolutions per second - 30 Hz-). Some embodiments may use the motor phase lag as detected in PLL 515 as a signal transmitted to communication protocol circuit 218. Thus, as the load on the motor changes, a phase lag or lead between the actual motor phase (or rotational position, as determined by motor controller 410, cf. FIG. 4) and the desired motor phase is recorded by IVUS control system 106. In some embodiments PLL 515 may adjust a voltage provided to the motor to drive the phase lead/lag towards an optimum value so that motor 450 operates more efficiently.

Embodiments of an IVUS imaging system such as system 100 using synchronous PIM 104 provide a simple mechanical hardware by eliminating the encoder in a motor control circuit. Some embodiments also provide a stable motor speed, independent of load. By using a clock and timing circuit such as circuit 200 (cf. FIG. 2) or circuit 500 (cf. FIG. 5) the control electronics may be located in synchronous PIM 104.

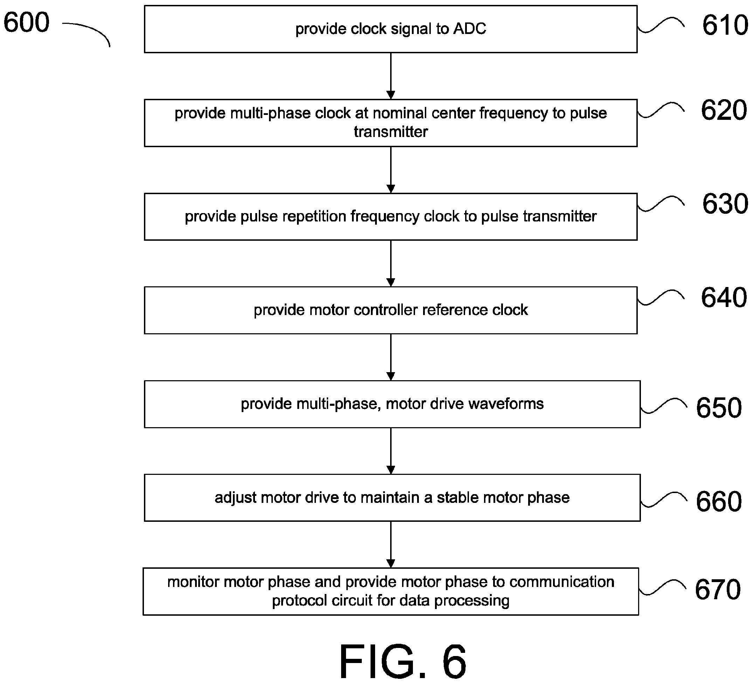

FIG. 6 shows a partial view of a flow chart of a method 600 for producing synchronized signals in a fire control system for rotational IVUS. Aspects of method 600 are performed by a PIM, such as synchronized PIM 104 (cf. FIG. 1), in some implementations.

Method 600 facilitates a decentralized system design, where the fire control and acquisition are positioned within the PIM such that the console hardware can be focused on signal processing and image display functions. With a timing scheme as disclosed herein, the transmit trigger timing and data acquisition circuitry can be devolved to the PIM hardware and a timing function de-centralized from IVUS control system 106 is provided (cf. FIG. 1). Embodiments consistent with the present disclosure provide an IVUS control system 106 as a centralized signal processing resource for a multi-modality system (*i.e*., with the modality-specific functions into modality-specific interfaces (*e.g*., PIM for IVUS) that are in communication with the control system 106). Accordingly, steps in method 600 may be partially performed by PIM 104, and by IVUS control system 106.

Step 610 includes providing a clock signal to an analog-to-digital converter circuit (e.g., signal 226 to ADC 216, cf. FIG. 2A). Step 620 includes providing a multi-phase clock signal at a nominal center frequency to a pulse transmitter (e.g., signal 222 to pulse transmitter 212, cf. FIG. 2A). Step 630 includes providing a pulse repetition frequency clock signal to the pulse transmitter (e.g., prf 522-2, cf. FIG. 5). Step 640 includes providing a motor controller reference clock signal to a motor controller circuit for a motor (e.g., signal 221 to motor control 210, cf. FIG. 2A). Step 650 includes providing multi-phase, motor drive waveforms to the motor controller circuit (e.g., waveforms 465, cf. FIG. 4). Step 660 includes adjusting the motor drive signal to maintain a stable motor phase. Step 670 includes monitoring a motor phase and providing the motor phase to a communication protocol circuit for data processing (e.g., circuit 218, cf. FIG. 2A).

Moving fire control and acquisition aspects into the PIM provides greater signal processing capability within the console 106, which can then be used to implement echo signal-based NURD reduction schemes that are very processor intensive. In that regard, NURD reduction algorithms combine with the high prf synchronous capability of embodiments consistent with method 600, as detailed below.

## Claims

1. A patient interface module (PIM) (104) for use in an intra-vascular ultrasound (IVUS) imaging system (100), the PIM comprising:
a motor (250, 450) having position sensors (455-1, 455-2, 455-3) adapted to provide a multi-phase waveform (465) for determining the position of a shaft (114, 414) included in said motor;
a motor controller circuit (210, 410) configured to provide a speed control signal (421, 221) to the motor;
a pulse transmitter circuit (212);
an analog-to-digital converter (ADC) circuit; and
a clock and timing circuit (200, 500) comprising a local oscillator (510) adapted to provide a trigger signal (222, 322) to said pulse transmitter circuit (212) and a digitizing signal (226) to said analog-to-digital converter (ADC) circuit and said speed control signal (221) to the motor controller circuit, the said signals being synchronized to said local oscillator (510), said digitizing signal being a signal for use by the ADC circuit to digitize electrical signals from a tissue echo;
wherein:
the motor is configured to provide a rotation signal (220, 320, 420, 324) to the motor controller circuit, said rotation signal including said multi-phase waveform, the motor controller circuit being adapted to provide said rotation signal to a data processing circuit (218) comprised in the patient interface module, said rotation signal providing a relative phase lead/ lag of said position of said shaft (114, 414) of said motor and the said trigger, speed control and digitizing signals.

2. The PIM of Claim 1, wherein the motor controller circuit is a synchronous motor controller circuit providing a multi-phase signal (521) to the motor.

3. The PIM of Claim 2 wherein the multiphase signal includes a plurality of waveforms evenly separated in phase over a cycle of the motor;
wherein each of the plurality of waveforms corresponds to a signal of a position sensor (455-1, -2, or -3) in the motor;
wherein the position encoder is selected from the group consisting of a magnetic sensor (455-1, -2, or -3) and an optical sensor (455-1, -2, or -3); and
wherein the magnetic sensor is a Hall effect sensor.

4. The PIM of Claim 2 wherein the synchronous motor controller circuit operates in an open loop configuration.

5. The PIM of Claim 1, wherein the motor controller circuit is a brushless DC motor controller (BLDC) circuit having a feedback loop to control a rotational speed of the motor.

6. The PIM of Claim 1 further comprising:
a receive amplifier (214) to provide an amplified echo signal in response to an acoustic pulse triggered by the pulse transmitter; and
a transducer (150) to provide an ultrasound signal triggered by the pulse transmitter and to provide an echo signal to the receive amplifier.

7. An imaging system (100) comprising:
a monitor (108);
a processing system (106);
a patient interface module (PIM) of any of the preceding claims; and
a catheter (102) coupled to the PIM, the catheter including a transducer (150).

8. The imaging system of Claim 7 wherein the processing system provides a two-dimensional (2D) image of a blood vessel tissue by arranging a plurality of A-scan lines (260) according to the relative phase value provided by the motor.

9. The imaging system of Claim 7, wherein the motor controller circuit is a synchronous motor controller circuit providing a multi-phase signal to the motor.

10. The imaging system of Claim 7 wherein the multiphase signal includes a plurality of waveforms (465) evenly separated in phase over a cycle of the motor.

11. A method for producing synchronized signals in a fire control system of a patient interface module for an intra-vascular ultrasound (IVUS) imaging system (100) having a transducer (150), a motor (250, 450) comprising position sensors (455-1, 455-2, 455-3) adapted to provide a multi-phase waveform (465) for determining the position of a shaft (114, 414) included in said motor, a motor controller circuit (210, 410) configured to provide a speed control signal (421, 221) to the motor, a pulse transmitter circuit (212), an analog-to-digital converter (ADC) circuit, and a clock and timing circuit (200, 500) comprising a local oscillator (510) adapted to provide a trigger signal (222, 322) to said pulse transmitter circuit (212) and a digitizing signal (226) to said analog-to-digital converter (ADC) circuit and said speed control signal (221) to the motor controller circuit, the said signals being synchronized to said local oscillator (510), said digitizing signal being a signal for use by the ADC circuit to digitize electrical signals from a tissue echo, the method comprising:
providing, with the motor, a rotation signal (220, 320, 420, 324) to the motor controller circuit, said rotation signal including said multi-phase waveform, the motor controller circuit being adapted to provide said rotation signal to a data processing circuit (218) comprised in the patient interface module, said rotation signal providing a relative phase lead/ lag of said position of said shaft (114, 414) of said motor and the said trigger, speed control and digitizing signals.

12. The method of Claim 11 further comprising adjusting an average motor speed to a pre-selected value.

13. The method of Claim 11 wherein the signal to the pulse transmitter comprises a multi-phase clock signal and a pulse repetition frequency.

14. The method of Claim 11 wherein the signal to the pulse transmitter comprises a signal at a frequency approximately equal to a center frequency in a response spectrum of an ultrasound transducer.

15. The method of Claim 14 further comprising:
receiving an ultrasound echo signal from the ultrasound transducer, the ultrasound echo signal originating form a target tissue;
forming a plurality of A-scans from the target tissue with the ultrasound echo signal; and
forming a two-dimensional (2D) image of a target tissue using the plurality of A-scans and the motor phase.

## Patentansprüche

1. Patientenschnittstellenmodul (PIM) (104) zur Verwendung in einem intravaskulären Ultraschall (IVUS) Bildgebungssystem (100), wobei das PIM umfasst:
einen Motor (250, 450), der Positionssensoren (455-1, 455-2, 455-3) aufweist, die ausgeführt sind, um eine Mehrphasen-Wellenform (465) zum Bestimmen der Position einer Welle (114, 414) bereitzustellen, die in dem Motor beinhaltet ist;
eine Motorsteuerschaltung (210, 410), konfiguriert, um ein Geschwindigkeitssteuersignal (421, 221) für den Motor bereitzustellen;
eine Impulsgeberschaltung (212);
eine Analog-Digital-Wandler (ADC) Schaltung; und
eine Uhren- und Taktschaltung (200, 500), einen lokalen Oszillator (510) umfassend, der ausgeführt ist, um ein Auslösesignal (222, 322) für die Impulsgeberschaltung (212) und ein Digitalisierungssignal (226) für die Analog-Digital-Wandler (ADC) Schaltung, und das Geschwindigkeitssteuersignal (221) für die Motorsteuerschaltung bereitzustellen, wobei die Signale zu dem lokalen Oszillator (510) synchronisiert werden, wobei das Digitalisierungssignal ein Signal zur Verwendung durch die ADC-Schaltung zum Digitalisieren elektrischer Signale aus einem Gewebeecho ist;
wobei:
der Motor konfiguriert ist, um ein Rotationssignal (220, 320, 420, 324) für die Motorsteuerschaltung bereitzustellen, wobei das Rotationssignal die Mehrphasen-Wellenform beinhaltet, die Motorsteuerschaltung ausgeführt ist, um das Rotationssignal für eine Datenverarbeitungsschaltung (218) bereitzustellen, die in dem Patientenschnittstellenmodul beinhaltet ist, wobei das Rotationssignal eine relative Phasen-Voreilung/ -Verzögerung der Position der Welle (114, 414) des Motors, und der Auslösungs-, der Geschwindigkeitssteuerungs- und Digitalisierungssignale bereitstellt.

2. PIM nach Anspruch 1, wobei die Motorsteuerschaltung eine Synchronmotorsteuerschaltung ist, die ein Mehrphasensignal (521) für den Motor bereitstellt.

3. PIM nach Anspruch 2, wobei das Mehrphasensignal eine Vielzahl von Wellenformen beinhaltet, die über einen Zyklus des Motors gleichförmig in der Phase getrennt sind;
wobei jede der Vielzahl von Wellenformen einem Signal eines Positionssensors (455-1, -2 oder -3) in dem Motor entspricht;
wobei der Positionsencoder aus der Gruppe ausgewählt wird, die aus einem Magnetsensor (455-1, -2 oder -3) und einem optischen Sensor (455-1, -2 oder -3) besteht; und
wobei der Magnetsensor ein Halleffektsensor ist.

4. PIM nach Anspruch 2, wobei die Synchronmotorsteuerschaltung in einer offenen Schleifenkonfiguration betrieben wird.

5. PIM nach Anspruch 1, wobei die Motorsteuerschaltung eine bürstenlose DC-Motorsteuerschaltung (BLDC) ist, die eine Rückmeldeschleife aufweist, um eine Rotationsgeschwindigkeit des Motors zu steuern.

6. PIM nach Anspruch 1, weiter umfassend:
einen Empfangsverstärker (214), um ein verstärktes Echosignal als Reaktion auf einen akustischen Impuls bereitzustellen, der durch den Impulsgeber ausgelöst wird; und
einen Umformer (150), um ein Ultraschallsignal bereitzustellen, das durch den Impulsgeber ausgelöst wird, und um ein Echosignal für den Empfangsverstärker bereitzustellen.

7. Bildgebungssystem (100), umfassend:
einen Monitor (108);
ein Verarbeitungssystem (106);
ein Patientenschnittstellenmodul (PIM) nach einem der vorstehenden Ansprüche; und
einen Katheter (102), der an das PIM gekoppelt ist, wobei der Katheter einen Umformer (150) beinhaltet.

8. Bildgebungssystem nach Anspruch 7, wobei das Verarbeitungssystem ein zweidimensionales (2D) Bild eines Blutgefäßgewebes durch Anordnen einer Vielzahl von A-Scan-Linien (260) entsprechend dem relativen Phasenwert bereitstellt, der durch den Motor bereitstellt wird.

9. Bildgebungssystem nach Anspruch 7, wobei die Motorsteuerschaltung eine Synchronmotorsteuerschaltung ist, die ein Mehrphasensignal für den Motor bereitstellt.

10. Bildgebungssystem nach Anspruch 7, wobei das Mehrphasensignal eine Vielzahl von Wellenformen (465) beinhaltet, die über einen Zyklus des Motors gleichförmig in der Phase getrennt sind.

11. Verfahren zum Erzeugen synchronisierter Signale in einem Feuerleitsystem eines Patientenschnittstellenmoduls für ein intravaskuläres Ultraschall (IVUS) Bildgebungssystem (100), das einen Umformer (150), einen Motor (250, 450), der Positionssensoren (455-1, 455-2, 455-3) umfasst, die ausgeführt sind, um eine Mehrphasen-Wellenform (465) zum Bestimmen der Position einer Welle (114, 414) bereitzustellen, die in dem Motor beinhaltet ist, eine Motorsteuerschaltung (210, 410), konfiguriert, um ein Geschwindigkeitssteuersignal (421, 221) für den Motor bereitzustellen, eine Impulsgeberschaltung (212), eine Analog-Digital-Wandler (ADC) Schaltung und eine Uhren- und Taktschaltung (200, 500) aufweist, einen lokalen Oszillator (510) umfassend, der ausgeführt ist, um ein Auslösesignal (222, 322) für die Impulsgeberschaltung (212) und ein Digitalisierungssignal (226) für die Analog-Digital-Wandler (ADC) Schaltung, und das Geschwindigkeitssteuersignal (221) für die Motorsteuerschaltung bereitzustellen, wobei die Signale zu dem lokalen Oszillator (510) synchronisiert werden, wobei das Digitalisierungssignal ein Signal zur Verwendung durch die ADC-Schaltung zum Digitalisieren elektrischer Signale aus einem Gewebeecho ist, wobei das Verfahren umfasst:
Bereitstellen, mit dem Motor, eines Rotationssignals (220, 320, 420, 324) an die Motorsteuerschaltung, wobei das Rotationssignal die Mehrphasen-Wellenform beinhaltet, die Motorsteuerschaltung ausgeführt ist, um das Rotationssignal an eine Datenverarbeitungsschaltung (218) bereitzustellen, der in dem Patientenschnittstellenmodul beinhaltet ist, wobei das Rotationssignal eine relative Phasen-Voreilung/ -Verzögerung der Position der Welle (114, 414) des Motors, und der Auslösungs-, der Geschwindigkeitssteuerungs- und Digitalisierungssignale bereitstellt.

12. Verfahren nach Anspruch 11, das weiter das Anpassen einer durchschnittlichen Motorgeschwindigkeit auf einen zuvor ausgewählten Wert umfasst.

13. Verfahren nach Anspruch 11, wobei das Signal an den Impulsgeber ein Mehrphasen-Uhrensignal und eine Impulswiederholungsfrequenz umfasst.

14. Verfahren nach Anspruch 11, wobei das Signal an den Impulsgeber ein Signal auf einer Frequenz annähernd gleich einer Mittenfrequenz in einem Reaktionsspektrum eines Ultraschallwandlers umfasst.

15. Verfahren nach Anspruch 14, weiter umfassend:
Empfangen eines Ultraschallechosignals von dem Ultraschallwandler, wobei das Ultraschallechosignal aus einem Zielgewebe stammt;
Bilden einer Vielzahl von A-Scans aus dem Zielgewebe mit dem Ultraschallechosignal; und
Bilden eines zweidimensionalen (2D) Bildes eines Zielgewebes unter Verwendung der Vielzahl von A-Scans und der Motorphase.

## Revendications

1. Module interface patient (MIP) (104) pour utilisation dans un système d'imagerie ultrasonore intravasculaire (USIV) (100), le MIP comprenant :
un moteur (250, 450) possédant des capteurs de position (455-1, 455-2, 455-3) adaptés pour fournir une forme d'onde multi-phase (465) pour déterminer la position d'un arbre (114, 414) inclus dans ledit moteur ;
un circuit de commande du moteur (210, 410) configuré pour fournir un signal de commande de vitesse (421, 221) au moteur ;
un circuit transmetteur d'impulsions (212) ;
un circuit convertisseur analogique-numérique (ADC) ; et
une horloge et un circuit de synchronisation (200, 500) comprenant un oscillateur local (510) adaptés pour fournir un signal de déclenchement (222, 322) audit circuit transmetteur d'impulsions (212) et un signal de numérisation (226) audit circuit convertisseur analogique-numérique (ADC) et ledit signal de commande de vitesse (221) au circuit de commande du moteur, lesdits signaux étant synchronisés avec ledit oscillateur local (510), ledit signal de numérisation étant un signal pour utilisation par le circuit ADC pour numériser les signaux électriques à partir d'un écho d'un tissu ;
dans lequel :
le moteur est configuré pour fournir un signal de
rotation (220, 320, 420, 324) au circuit de commande du moteur, ledit signal de rotation incluant ladite forme d'onde multi-phase, le circuit de commande du moteur étant adapté pour fournir ledit signal de rotation à un circuit de traitement de données (218) compris dans le module interface patient, ledit signal de rotation fournissant une avance/un retard de phase relative de ladite position dudit arbre (114, 414) dudit moteur et lesdits signaux de déclenchement, de commande de vitesse et de numérisation.

2. MIP selon la revendication 1, dans lequel le circuit de commande du moteur est un circuit de commande du moteur synchrone fournissant un signal multi-phase (521) au moteur.

3. MIP selon la revendication 2 dans lequel le signal multi-phase inclut une pluralité de formes d'onde séparées de manière régulière en phase sur un cycle du moteur ;
dans lequel chacune de la pluralité de formes d'onde correspond à un signal d'un capteur de position (455-1, -2, ou -3) dans le moteur ;
dans lequel le codeur de position est choisi dans le groupe constitué d'un capteur magnétique (455-1, -2, ou -3) et d'un capteur optique (455-1, -2, ou -3) ; et
dans lequel le capteur magnétique est un capteur à effet Hall.

4. MIP selon la revendication 2 dans lequel le circuit de commande du moteur synchrone fonctionne dans une configuration de boucle ouverte.

5. MIP selon la revendication 1, dans lequel le circuit de commande du moteur est un circuit de commande de moteur à courant continu sans balai (BLDC) possédant une boucle d'asservissement pour commander une vitesse de rotation du moteur.

6. MIP selon la revendication 1 comprenant en outre :
un récepteur amplificateur (214) pour fournir un signal d'écho amplifié en réponse à une impulsion acoustique déclenchée par le transmetteur d'impulsions ; et
un transducteur (150) pour fournir un signal ultrasonore déclenché par le transmetteur d'impulsions et pour fournir un signal d'écho au récepteur amplificateur.

7. Système d'imagerie (100) comprenant :
un moniteur (108) ;
un système de traitement (106) ;
un module interface patient (MIP) selon l'une quelconque des revendications précédentes ; et
un cathéter (102) couplé au MIP, le cathéter incluant un transducteur (150).

8. Système d'imagerie selon la revendication 7 dans lequel le système de traitement fournit une image bidimensionnelle (2D) d'un tissu de vaisseau sanguin par agencement d'une pluralité de lignes d'échographie A (260) selon la valeur de phase relative fournie par le moteur.

9. Système d'imagerie selon la revendication 7, dans lequel le circuit de commande du moteur est un circuit de commande du moteur synchrone fournissant un signal multi-phase au moteur.

10. Système d'imagerie selon la revendication 7 dans lequel le signal multi-phase inclut une pluralité de formes d'onde (465) séparées de manière régulière en phase sur un cycle du moteur.

11. Procédé de production de signaux synchronisés dans un système de commande de tir d'un module interface patient pour un système d'imagerie ultrasonore intravasculaire (USIV) (100) possédant un transducteur (150), un moteur (250, 450) comprenant des capteurs de position (455-1, 455-2, 455-3) adaptés pour fournir une forme d'onde multi-phase (465) pour déterminer la position d'un arbre (114, 414) inclus dans ledit moteur, un circuit de commande du moteur (210, 410) configuré pour fournir un signal de commande de vitesse (421, 221) au moteur, un circuit transmetteur d'impulsions (212), un circuit convertisseur analogique-numérique (ADC), et une horloge et un circuit de synchronisation (200, 500) comprenant un oscillateur local (510) adaptés pour fournir un signal de déclenchement (222, 322) audit circuit transmetteur d'impulsions (212) et un signal de numérisation (226) audit circuit convertisseur analogique-numérique (ADC) et ledit signal de commande de vitesse (221) au circuit de commande du moteur, lesdits signaux étant synchronisés avec ledit oscillateur local (510), ledit signal de numérisation étant un signal pour utilisation par le circuit ADC pour numériser les signaux électriques à partir d'un écho d'un tissu, le procédé comprenant :
la fourniture, avec le moteur, d'un signal de rotation (220, 320, 420, 324) au circuit de commande du moteur, ledit signal de rotation incluant ladite forme d'onde multi-phase, le circuit de commande du moteur étant adapté pour fournir ledit signal de rotation à un circuit de traitement de données (218) compris dans le module interface patient, ledit signal de rotation fournissant une avance/un retard de phase relative de ladite position dudit arbre (114, 414) dudit moteur et lesdits signaux de déclenchement, de commande de vitesse et de numérisation.

12. Procédé selon la revendication 11 comprenant en outre l'ajustement d'une vitesse moyenne du moteur à une valeur présélectionnée.

13. Procédé selon la revendication 11 dans lequel le signal au transmetteur d'impulsions comprend un signal d'horloge multi-phase et une fréquence de répétition d'impulsions.

14. Procédé selon la revendication 11 dans lequel le signal au transmetteur d'impulsions comprend un signal à une fréquence approximativement égale à une fréquence centrale dans un spectre de réponse d'un transducteur ultrasonore.

15. Procédé selon la revendication 14 comprenant en outre :
la réception d'un signal d'écho ultrasonore à partir du transducteur ultrasonore, le signal d'écho ultrasonore provenant d'un tissu cible ;
la formation d'une pluralité d'échographies A à partir du tissu cible avec le signal d'écho ultrasonore ; et
la formation d'une image bidimensionnelle (2D) d'un tissu cible en utilisant la pluralité d'échographies A et la phase du moteur.
